# FASCICULE DE BREVET EUROPEEN

(11) **EP 0 681 827 B1**
(45) Date de publication et mention de la délivrance du brevet: **22.01.1997**
(21) Numéro de dépôt: 95400787.8
(22) Date de dépôt: 07.04.1995
(51) Int. Cl.: A61K 7/06, A61K 7/04, A61K 7/043, A61K 7/032

(54) **Utilisations de bioflavonoides comme agent de protection des phanères kératiniques**
Verwendung von Bioflavonoiden als Schutzmittel für Keratinmaterialien
Use of bioflavonoids as protecting agent for keratin materials

(30) Priorité: 05.05.1994 FR 9405540
(43) Date de publication de la demande: 15.11.1995
(73) Titulaire: L'OREAL, 75008 Paris (FR)
(72) Inventeur: Dubief, Claude, F-78150 Le Chesnay (FR); Braida-Valerio, Damarys, F-75004 Paris (FR); Cauwet, Danièle, F-75011 Paris (FR)
(74) Mandataire: Tezier Herman, Béatrice

(56) Documents cités:
- EP-A- 0 461 827
- CH-A- 559 547
- DE-A- 1 910 561
- FR-A- 2 207 699
- FR-A- 2 578 165
- FR-A- 2 666 226
- FR-A- 2 699 818
- S.T.N., Serveur de bases de données, Karlsruhe, DE, Fichier Chemical Abstracts, vol 118, n 45487 * résumé * & JP-A-04266809 (POLA CHEM.)
- S.T.N., Serveur de bases de données, Karlsruhe, DE, Fichier Chemical Abstracts, vol 122, n 178420, * résumé * & JP-A-07002677 (TOKYO SUGAR REF.)
- PATENT ABSTRACTS OF JAPAN vol. 17, no. 291 (C-1067) & JP-A-05 017 321 (GORO KAWAGUCHI)
- PATENT ABSTRACTS OF JAPAN vol. 15, no. 112 (C-815) & JP-A-03 005 423 (ICHIMARU PHARCOS CO)
- S.T.N., Serveur de bases de données, Karlsruhe, DE, Fichier Chemical Abstracts, vol 118, n 87384, * résumé * & JP-A-04275210 ( TSUKADA )
- PATENT ABSTRACTS OF JAPAN vol. 12, no. 297 (C-519) (3144) & JP-A-63 066 110 (MOMOTANI JIYUNTENKAN)
- PATENT ABSTRACTS OF JAPAN vol. 16, no. 164 (C-931) & JP-A-04 013 691 (HAYASHIBARA BIOCHEM LAB INC)

## Description

La présente invention concerne l'utilisation de certains bioflavonoïdes comme agents de traitement dans des compositions cosmétiques dans un but de protection des phanères kératiniques tels qu'en particulier les cheveux, les cils ou les ongles. L'invention a également pour objet un procédé visant à la protection de la kératine de ces phanères, en particulier contre les différentes dégradations susceptibles d'être occasionnées à cette dernière par des agressions de type atmosphériques ou autres. Elle concerne également des compositions stables contenant certains bioflavonoïdes.

Par phanères kératiniques, on comprendra plus particulièrement les cheveux, les cils, les sourcils et les ongles. L'utilisation et les compositions selon la présente invention sont particulièrement destinées aux cheveux.

Il est bien connu que les phanères kératiniques tels que les cheveux, les cils ou les ongles sont sensibilisés ou fragilisés à des degrés divers par l'action des agents atmosphériques et de la lumière, ainsi que par l'action répétée de différents traitements plus ou moins aggressifs tels que les permanentes, le défrisage, la teinture, la décoloration, les lavages et autres. Les cheveux deviennent alors rêches au toucher, sont difficiles à démêler et à coiffer en outre, les propriétés mécaniques des phanères kératiniques comme la résistance à la traction, la charge de rupture et l'élasticité, sont à la longue altérées.

Pour lutter contre les dégradations de la kératine des cheveux par la lumière, on a déjà proposé d'utiliser certaines substances susceptibles de filtrer les radiations lumineuses, comme l'acide 2-hydroxy-4-méthoxy benzophénone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

Par ailleurs, certains flavonoïdes sont connus pour leur utilisation dans la préparation de compositions cosmétiques, à titre d'agents de protection de la peau et/ou de ses phanères kératiniques contre l'oxygène singulet, tel que cela est décrit dans la demande de brevet FR-A-2 687 752. Toutefois, non seulement ces flavonoïdes ne correspondent pas à ceux mis en oeuvre dans le cadre de la présente invention, mais en outre ce document reste muet sur les effets particuliers qui pourraient être obtenus par application desdits flavonoïdes sur les cheveux, les cils ou les ongles.

*Le résumé de la demande JP04266809 décrit une composition pour les ongles contenant des flavonoïdes (hespéridine).*

*Le document CH559 547 décrit une composition cosmétique contenant un complexe biologique phyto-aromatique et du myristate d'isopropyle. Le complexe biologique contient des essences de citrus, de romarin et de thym. Parmi les nombreux constituants de ces essences, on cite l'hespéridine.*

*Le résumé de la demande JP05017321 décrit une composition pour la croissance des cheveux contenant de la vitamine P (hespéridine) et de la vitamine C.*

Or, la demanderesse a maintenant découvert, de façon inattendue et surprenante, que certains flavonoïdes spécifiques, tels que définis ci-après, ont une action renforçatrice sur la kératine des phanères et ont un effet protecteur vis à vis de la dégradation des phanères kératiniques par la lumière. En outre, ils apportent d'excellentes propriétés cosmétiques aux phanères kératiniques, en particulier en améliorant en particulier le démêlage et/ou le coiffage et/ou la douceur.

La demanderesse a constaté que les compositions contenant des bioflavonoides ne sont pas très stables. En effet, les compositions se colorent fortement, elles deviennent jaunes ou brunes. L'efficacité des bioflavonoides peut également diminuer.

La demanderesse a maintenant découvert qu'en ajoutant un agent réducteur particulier, les compositions contenant des bioflavonoides étaient stables et ne présentaient pas les inconvénient sus-mentionnés.

Ces découvertes sont à la base de la présente invention.

Dans l'un de ses premiers aspects, la présente invention a ainsi pour objet l'utilisation dans une composition cosmétique d'au moins un bioflavonoïde de citrus,
comme agent de traitement visant à la protection ou à l'amélioration des propriétés physiques, en particulier des propriétés mécaniques et/ou des propriétés cosmétiques des phanères kératiniques.

Les propriétés mécaniques améliorées sont plus particulièrement la résistance à la traction, la charge de rupture ou l'élasticité.
Les propriétés cosmétiques sont, en particulier, le démêlage et/ou le coiffage et/ou la douceur.

Les bioflavonoïdes de citrus sont présents dans les écorces de citrus tels que le citron, l'orange, la mandarine ou le pamplemousse. lls sont connus également pour leur aptitude à maintenir en bon état les vaisseaux sanguins par diminution de la fragilité et de la perméabilité des vaisseaux capillaires (The Merck Index; 1989; page 1243).

Selon un autre aspect de la présente invention, les bioflavonoïdes utilisables dans le cadre de la présente invention sont choisis parmi les composés de formule (I) : 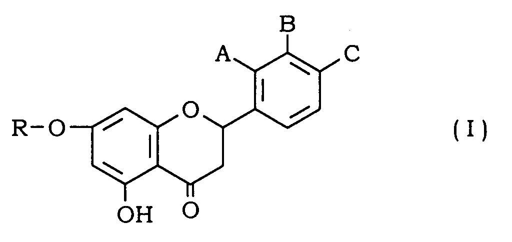
dans laquelle
- R représente un radical d'un sucre
- A représente un atome d'hydrogène ou un radical alcoxy ayant environ de 1 à 4 atomes de carbone,
- B représente un atome d'hydrogène ou un radical hydroxy ou alcoxy ayant environ de 1 à 4 atomes de carbone,
- C représente un atome d'hydrogène ou un radical hydroxy ou alcoxy ayant environ de 1 à 4 atomes de carbone,
ou de formule (II) : 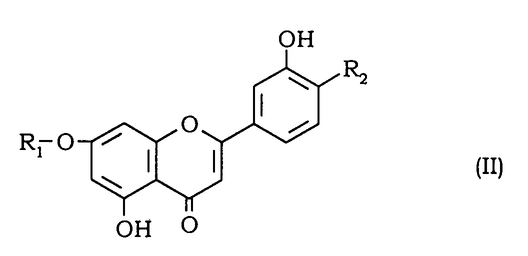
dans laquelle R₁ désigne un radical 6-O-(6-déoxy-α-L-mannopyranosyl)-β-D-glucopyranosyl et R₂ désigne un radical alcoxy ayant environ de 1 à 4 atomes de carbone.

Comme précédemment, l'invention concerne également, selon un autre aspect de cette dernière, l'utilisation dans une composition cosmétique, d'au moins un bioflavonoïde tel que défini par les formules (I) ou (II) ci-dessus,
comme agent de traitement visant à la protection ou à l'amélioration des propriétés physiques, en particulier des propriétés mécaniques et/ou des propriétés cosmétiques des phanères kératiniques.

Les propriétés mécaniques améliorées sont plus particulièrement la résistance à la traction, la charge de rupture ou l'élasticité.
Les propriétés cosmétiques sont, en particulier, le démêlage et/ou le coiffage et/ou la douceur.

La présente invention a aussi pour objet une composition cosmétique contenant au moins un bioflavonoïde de citrus et/ou au moins un bioflavonoïde tel que défini par les formules (I) ou (II) ci-dessus, et au moins un agent réducteur choisi parmi les sels alcalins ou alcalinoterreux de métabisulfite, l'acide érythorbique et la cystéine.

Dans le cadre de la présente invention, A représente, de préférence, un atome d'hydrogène ou un radical méthoxy, B et C représentent , de préférence et indépendemment l'un de l'autre, un atome d'hydrogène ou un radical hydroxy ou méthoxy, R₂ représente de préférence un radical méthoxy. R représente, de préférence, un reste choisi parmi les groupements 6-O-(6-déoxy-α-L-mannopyranosyl)-β-D-glucopyranosyl, 2-O-(6-déoxy-α-L-mannopyranosyl)-β-D-glucopyranosyl et 6-déoxy-α-L-mannopyranosyl.

Les composés de formule (I) encore plus particulièrement préférés sont choisis parmi la naringine, la néohespéridine, l'hespéridine et l'ériodictine.

Les composés de formule (I) et (II) sont des bioflavonoïdes qui sont extraits de plantes et peuvent être également obtenus selon les procédés décrits dans "The Flavonoïds" Harbone J.B., Mabry T.J., Helga Mabry, 1975.

Les bioflavonoïdes utilisés selon l'invention possèdent une bonne affinité vis à vis des phanères kératiniques. Ils renforcent les propriétés physiques des phanères kératiniques, notamment contre la dégradation par la lumière. Ils préservent les propriétés mécaniques des phanères kératiniques, et notamment leur résistance à la traction, leur élasticité, leur vitesse de gonflement dans un milieu aqueux. Les cheveux ainsi traités présentent de bonnes propriétés cosmétiques notamment au niveau de leur facilité à être démêlés. Les ongles se dédoublent moins.

Les composés de formule (I) et (II) sont présents dans des extraits d'agrumes et en particulier de citron. De tels extraits sont notamment commercialisés par la société INTERCHEMICAL sous les dénominations "CITRUS BIOFLAVONOÏD COMPLEX 45%", "LEMON BIOFLAVONOÏD COMPLEX 50%", "GRAPEFRUIT BIOFLAVONOÏD COMPLEX 25% et "ORANGE BIOFLAVONOÏD COMPLEX 25%".

Dans les compositions selon l'invention et pour l'utilisation selon l'invention, les composés de formule (I) et (II) et/ou les bioflavonoïdes de citrus sont généralement présents à une concentration comprise entre 0,001 et 10 % en poids et de préférence entre 0,005 et 5 % en poids par rapport au poids total de la composition.

L'agent réducteur est de préférence choisi parmi les sels de métabisulfite et l'acide érythorbique.

Les sels de métabisulfite sont de préférence les sels de sodium et de potassium.

L'agent réducteur est présent dans des concentrations allant de 0,001 à 3% en poids par rapport au poids total de la composition et de préférence de 0,05 à 1% en poids.

Les compositions peuvent se présenter sous forme de lotion aqueuse ou hydroalcoolique, monophasique ou polyphasique, de gel monophasique ou polyphasique, d'émulsion, de crème, de dispersion vésiculaire, de mousse, de spray.

Les compositions utilisées selon l'invention sont par exemple des composition capillaires rincées ou non rincées, des compositions de soin et/ou de maquillage des cils ou des sourcils telles que les mascaras, des compositions de soin pour les ongles, ou des vernis à ongle.

Les compositions capillaires peuvent se présenter sous forme de shampooing, d'après-shampooing à rincer ou non, de mousse de coiffage, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs tels que des protéines, des vitamines, les agents traitants (agents anti-chute, antipelliculaires), les céramides tels que ceux cités dans EP-A-500 437, les colorants, les agents nacrants, les filtres solaires et notamment les filtres sulfoniques, les parfums, les conservateurs, les agents antimicrobiens, les électrolytes, les agents séquestrants, les agents propulseurs.

Plus précisément, comme corps gras, on peut utiliser une huile ou une cire ou leur mélange, des acides gras, des alcools gras, des esters d'acides gras tels que les triglycérides d'acide gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone, les isoparaffines et les huiles fluorées ou perfluorées.

Parmi les cires, on peut citer les cires animales, végétales, minérales ou de synthèse, et notamment les cires d'abeilles, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, l'éthylèneglycol, le diéthylènegycol, le propylèneglycol, le glycérol.

Les agents épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, la gomme de xanthane, les scléroglucanes, les acides polyacryliques réticulés.

Comme agents tensio-actifs et comme polymères, on peut utiliser tous ceux bien connus de l'état de la technique notamment pour leur utilisation dans des compositions cosmétiques.

Bien entendu, l'homme de l'art veillera à choisir les éventuels additifs de manière telle que les propriétés de la composition ne soient pas, ou substantiellement pas altérées par ces additifs.

Les compositions peuvent se présenter sous forme de dispersion vésiculaire de lipides amphiphiles ioniques ou non-ioniques. Elles sont alors préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 3 et 9 et de préférence inférieur à 6, et encore plus particulièrement compris entre 3 et 5.

Les composés de formule (I) et (II) peuvent être ajoutés à la composition juste avant l'emploi ; ils sont donc , dans ce cas, conditionnés à part des autres ingrédients de la composition.

L'invention a aussi pour objet un procédé de protection de la kératine des phanères, en particulier contre les agressions atmosphériques, et notamment la lumière, consistant à appliquer sur ces phanères kératiniques à protéger une quantité efficace d'au moins un composé de formule (I) et/ou (II) et/ou d'un bioflavonoïde de citrus tels que définis ci-dessus, dans un support cosmétiquement acceptable, cette application étant éventuellement suivie d'un rinçage à l'eau.

On va maintenant donner à titre d'illustration et sans aucun caractère limitatif plusieurs exemples conformes à l'invention.

### EXEMPLE 1

On a préparé un après-shampooing de composition suivante :

| | |
|---|---|
| - alcool cérylstéarylique | 2, 8 g |
| - mono et distéarate de glycérol | 0, 5 g |
| - lanoline | 0, 5 g |
| - chlorure de cétyltriméthylammonium en solution aqueuse à 25 % vendu sous la dénomination "ARQUAD 16-25" par la société AKZO | 0, 55 g (MA) |
| - mélange de bioflavonoïdes^{**(1)**} vendu sous la dénomination "grapefruit Bioflavonoïd complex 25 %" par la société INTERCHEMICAL | 0, 5 g |
| - conservateurs | qs |
| - parfums | qs |
| - eau qsp | 100 g |
| pH spontané : 4,6 | |

| | |
|---|---|
| ^{**(1)**} : l'extrait contient 21,5 % en poids de naringine, 0,7 % d'hespéridine et 0,7 % de néohespéridine. | |

Cet après-shampooing a été appliqué sur des cheveux mouillés après un simple shampooing. Après rinçage à l'eau, puis séchage, les cheveux étaient lisses, faciles à démêler et présentait une très bonne douceur.

### EXEMPLE 2

On a préparé une composition capillaire de soin non rincée ayant la composition suivante :

| | |
|---|---|
| - copolymère d'acrylamide et du sel sulfonique d'acrylamido-2-méthyl propane sulfonique en émulsion inverse à 40 % dans un mélange isoparaffine/eau vendu sous la dénomination "SEPIGEL 305" par la société SEPPIC | 1,2 g (MA) |
| - mélange de polydiméthylsiloxane α,ω dihydroxylé, de cyclotétradiméthylsiloxane et de cyclopentadiméthylsiloxane vendu sous la dénomination "Q₂ 1401" par la société DOW CORNING | 20 g |
| - mélange de bioflavonoïdes^{**(2)**} vendu sous la dénomination "citrus Bioflavonoïd complex 45%" par la société INTERCHEMICAL | 0,1 g |
| - conservateurs | qs |
| - parfums | qs |
| - eau qsp | 100 g |
| pH spontané : 6,3 | |

| | |
|---|---|
| ^{**(2)**} : l'extrait contient 23, 95% en poids de naringine, 11, 45% de néohespéridine, 1, 24% de diosmine et 0, 40% d'hespéridine. | |

Cette composition de soin a été appliquée sur des cheveux lavés, sans rinçage. Après séchage, les cheveux étaient faciles à démêler et présentaient un très bon coiffant.

### EXEMPLE 3

On a préparé un shampooing ayant la composition suivante :

| | |
|---|---|
| - lauryl éther sulfate de sodiuml et de magnésium(80/20) oxyéthyléné à 2,2 moles d'oxyde d'éthylène en solution aqueuse à 26 % en poids vendu sous la dénomination "EMPICOL BSD" par la Société ALBRIGHT & WILSON | 10 g (MA) |
| - mélange cocoylamidopropylbetaïne/monolaurate de glycérol (25/5) vendu sous la dénomination "TEGOBETAÏNE HS" par la société GOLDSCHMIDT à 30% de matière active | 5 g MA |
| - mélange de bioflavonoïdes vendu sous la dénomination "citrus Bioflavonoïd complex 45%" par la société INTERCHEMICAL | 0,5 g |
| - acide érythorbique | 0,5 g |
| - parfums | qs |
| - conservateurs | qs |
| - eau qsp | 100 g |

Ce shampooing a été appliqué sur des cheveux mouillés: il présente de bonnes propriétés moussantes et permet de protéger efficacement les cheveux contre l'action de la lumière.

### EXEMPLE 4

On a préparé un mascara conforme à l'invention de composition suivante :

| Phase A : | |
|---|---|
| - Cire d'abeille | 6,9 g |
| - Cire de carnauba | 4,16 g |
| - Paraffine | 11,4 g |
| - Acide stéarique | 7,7 g |

| Phase B : | |
|---|---|
| - Oxyde de fer noir | 5,55 g |

| Phase C: | |
|---|---|
| - Triéthanolamine | 3,8 g |
| - Gomme arabique | 4,5 g |
| - Mélange de bioflavonoïdes^{**(2)**} vendu sous la dénomination "citrus Bioflanonoïd complex 45%" par la société INTERCHEMICAL | 0,1 g |
| - Conservateurs | qs |
| - Eau déminéralisée qs | 100 g |

On fait fondre la phase A à 80°C puis on introduit la phase B que l'on disperse à l'aide d'un homogénéïsateur pendant 30 minutes.
On prépare la phase C en introduisant les trois premiers composants de cette phase dans l'eau portée à 75°C.
On réalise ensuite une émulsion en mélangeant la phase C à la phase A+B .

On a appliqué la composition de mascara sur les cils. Les cils présentent une bonne tenue.

## Revendications

1. Utilisation cosmétique dans une composition cosmétique d'au moins un bioflavonoïde de citrus et/ou d'un bioflavonoïde choisi parmi les composés de formule (I) : 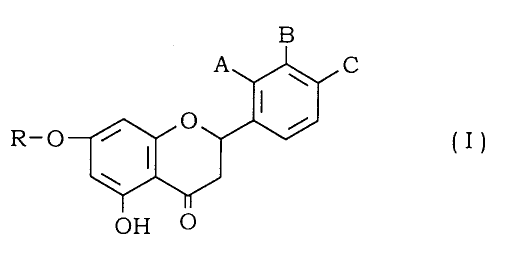 dans laquelle
- R représente un radical d'un sucre,
- A représente un atome d'hydrogène ou un radical alcoxy ayant de 1 à 4 atomes de carbone,
- B représente un atome d'hydrogène ou un radical hydroxy ou alcoxy ayant de 1 à 4 atomes de carbone,
- C représente un atome d'hydrogène ou un radical hydroxy ou alcoxy ayant de 1 à 4 atomes de carbone,
ou de formule (II) : 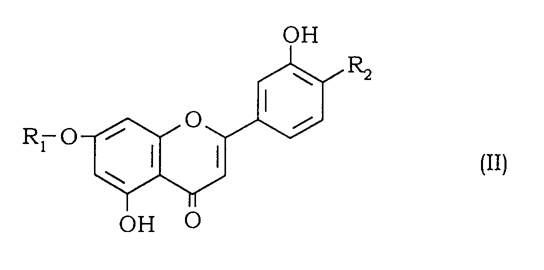 dans laquelle R₁ désigne un radical 6-O-(6-déoxy-α-L-mannopyranosyl)-β-D-glucopyranosyl et R₂ désigne un radical alcoxy ayant de 1 à 4 atomes de carbones,
comme agent de traitement visant à la protection ou à l'amélioration des propriétés physiques, en particulier des propriétés mécaniques et/ou des propriétés cosmétiques des phanères kératiniques.

2. Utilisation selon la revendication 1, caractérisée en ce que A représente un atome d'hydrogène ou un radical méthoxy, B représente un atome d'hydrogène ou un radical hydroxy ou méthoxy, C représente un atome d'hydrogène ou un radical hydroxy ou méthoxy et R₂ représente un radical méthoxy.

3. Utilisation selon l'une quelconque des revendications 1 ou 2, caractérisée en ce que R est un radical choisi parmi les groupements 6-O-(6-déoxy-α-L-mannopyranosyl)-β-D-glucopyranosyl, 2-O-(6-déoxy-α-L-mannopyranosyl)-β-D-glucopyranosyl et 6-déoxy-α-L-mannopyranosyl.

4. Utilisation selon l'une quelconque des revendications précédentes, caractérisée en ce que le composé de formule (I) est choisi parmi la naringine, la néohespéridine, l'hespéridine et l'ériodictine.

5. Utilisation selon l'une quelconque des revendications précédentes, caractérisée en ce que les bioflavonoïdes sont présents à une concentration comprise entre 0, 001 % et 10 % et de préférence entre 0, 005 et 5 % en poids par rapport au poids total de la composition.

6. Utilisation selon l'une quelconque des revendications précédentes, caractérisée en ce que la composition cosmétique se présente sous forme de lotion aqueuse ou hydroalcoolique, de gel, de crème, d'émulsion, de dispersion vésiculaire, de mousse, de spray.

7. Utilisation selon l'une quelconque des revendications précédentes, caractérisée en ce que les propriétés cosmétiques améliorées sont le démêlage et/ou le coiffage et/ou la douceur.

8. Utilisation selon l'une quelconque des revendications 1 à 7, caractérisée en ce que les propriétés mécaniques améliorées sont la résistance à la traction, la charge de rupture ou l'élasticité.

9. Utilisation selon l'une quelconque des revendications précédentes, caractérisée en ce que les phanères kératiniques sont des cheveux.

10. Utilisation selon l'une quelconque des revendications précédentes, caractérisée en ce que la composition cosmétique est une composition capillaires rincée ou non rincée.

11. Utilisation selon la revendication 10, caractérisée en ce que la composition cosmétique capillaire se présente sous forme de shampooing, d'après-shampooing à rincer ou non, de composition pour permanente, défrisage, coloration ou décoloration, ou encore sous forme de composition à rincer, à appliquer avant ou après une coloration, une permanente ou un défrisage ou encore entre les deux étapes d'une permanente ou d'un défrisage.

12. Utilisation selon l'une quelconque des revendications 1 à 8, caractérisée en ce que les phanères kératiniques sont des cils ou des sourcils.

13. Utilisation selon la revendication 12, caractérisée en ce que les compositions cosmétiques sont des compositions de soin ou de maquillage des cils ou des sourcils.

14. Utilisation selon l'une quelconque des revendications 1 à 8, caractérisée en ce que les phanères kératiniques sont des ongles.

15. Utilisation selon la revendication 14, caractérisée en ce que les compositions cosmétiques sont des compositions de soin pour les ongles ou des vernis à ongle.

16. Utilisation selon l'une quelconque des revendications précédentes, caractérisée en ce que la composition cosmétique contient au moins un additif cosmétique.

17. Composition cosmétique contenant au moins un bioflavonoïde de citrus et/ou au moins un bioflavonoïde tel que défini à l'une quelconque des revendications 1 à 4, caractérisée en ce qu'elle contient en outre au moins un agent réducteur choisi parmi les sels alcalins ou alcalinoterreux de métabisulfite, l'acide érythorbique et la cystéine.

18. Composition selon la revendication 17, caractérisée par le fait que l'agent réducteur est choisi parmi les sels de métabisulfite et l'acide érythorbique.

19. Composition selon l'une quelconque des revendications 17 ou 18, caractérisée par le fait que les sels de métabisulfites sont les sels de sodium et de potassium.

20. Composition selon l'une quelconque des revendications 17 à 19, caractérisée par le fait que le pH de la composition est inférieur à 6.

21. Composition selon l'une quelconque des revendications 17 à 20, caractérisée par le fait que le pH de la composition est compris entre 3 et 5.

22. Composition selon l'une quelconque des revendications 17 à 21, caractérisée par le fait que l'agent réducteur est présent dans des concentrations allant de 0,001 à 3% en poids par rapport au poids total de la composition.

23. Composition selon la revendication 22, caractérisée par le fait que l'agent réducteur est présent dans des concentrations allant de 0,005 à 1 % en poids par rapport au poids total de la composition.

24. Composition selon l'une quelconque des revendications 17 à 23, caractérisée en ce que les bioflavonoïdes sont présents à une concentration comprise entre 0,001 % et 10 % et de préférence entre 0,005 et 5 % en poids par rapport au poids total de la composition.

25. Composition selon la revendication 24, caractérisée en ce que les bioflavonoïdes sont présents à une concentration comprise entre 0,005 et 5 % en poids par rapport au poids total de la composition.

26. Composition selon l'une quelconque des revendications 17 à 25, caractérisée par le fait la composition cosmétique est telle que décrite dans l'une quelconque des revendications 6 à 16.

27. Utilisation cosmétique selon l'une quelconque des revendications 1 à 16, caractérisée par le fait que la composition est telle que définie à l'une quelconque des revendications 17 à 26.

## Claims

1. Cosmetic use, in a cosmetic composition, of at least one citrus bioflavonoid and/or one bioflavonoid chosen from the compounds of formula (I): 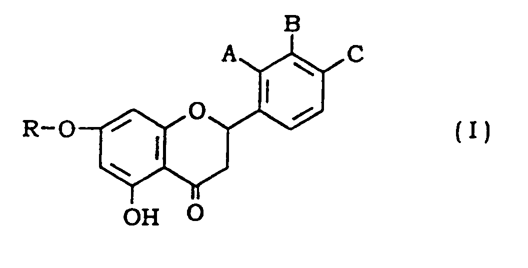 in which
- R represents a radical of a sugar,
- A represents a hydrogen atom or an alkoxy radical having from 1 to 4 carbon atoms,
- B represents a hydrogen atom or a hydroxyl or alkoxy radical having from 1 to 4 carbon atoms,
- C represents a hydrogen atom or a hydroxyl or alkoxy radical having from 1 to 4 carbon atoms,
or of formula (II): 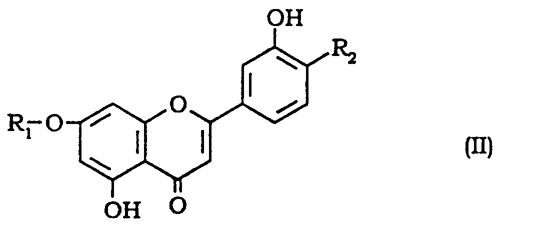 in which R₁ designates a 6-O-(6-deoxy-α-L-mannopyranosyl)-β-D-glucopyranosyl radical and R₂ designates an alkoxy radical having from 1 to 4 carbon atoms, as treatment agent for protecting or for improving the physical properties, in particular the mechanical properties, and/or the cosmetic properties of superficial keratinous body growths.

2. Use according to Claim 1, characterized in that A represents a hydrogen atom or a methoxy radical, B represents a hydrogen atom or a hydroxyl or methoxy radical, C represents a hydrogen atom or a hydroxyl or methoxy radical and R₂ represents a methoxy radical.

3. Use according to either of Claims 1 and 2, characterized in that R is a radical chosen from the 6-O-(6-deoxy-α-L-mannopyranosyl)-β-D-glucopyranosyl, 2-O-(6-deoxy-α-L-mannopyranosyl)-β-D-glucopyranosyl and 6-deoxy-α-L-mannopyranosyl groups.

4. Use according to any one of the preceding claims, characterized in that the compound of formula (I) is chosen from naringin, neohesperidin, hesperidin and eriodictin.

5. Use according to any one of the preceding claims, characterized in that the bioflavonoids are present at a concentration of between 0.001 % and 10 %, and preferably of between 0.005 and 5 % by weight relative to the total weight of the composition.

6. Use according to any one of the preceding claims, characterized in that the cosmetic composition is provided in the form of an aqueous or aqueous-alcoholic lotion, a gel, a cream, an emulsion, a vesicular dispersion, a foam or a spray.

7. Use according to any one of the preceding claims, characterized in that the improved cosmetic properties are the disentanglement and/or styling and/or softness.

8. Use according to any one of Claims 1 to 7, characterized in that the improved mechanical properties are the tensile strength, the breaking load or the elasticity.

9. Use according to any one of the preceding claims, characterized in that the keratinous superficial body growths are the hair.

10. Use according to any one of the preceding claims, characterized in that the cosmetic composition is a rinse-off or leave-in hair composition.

11. Use according to Claim 10, characterized in that the cosmetic hair composition is provided in the form of a shampoo, a rinse-off or leave-in conditioner, a composition for permanent waving, hair straightening, dyeing or bleaching, or alternatively in the form of a rinse-off composition, to be applied before or after dyeing, permanent waving or hair straightening or alternatively between the two stages of a permanent waving or a hair straightening.

12. Use according to any one of Claims 1 to 8, characterized in that the keratinous superficial body growths are eyelashes or eyebrows.

13. Use according to Claim 12, characterized in that the cosmetic compositions are treatment or make-up compositions for the eyelashes or the eyebrows.

14. Use according to any one of Claims 1 to 8, characterized in that the keratinous superficial body growths are nails.

15. Use according to Claim 14, characterized in that the cosmetic compositions are treatment compositions for the nails, or nail varnishes.

16. Use according to any one of the preceding claims, characterized in that the cosmetic composition contains at least one cosmetic additive.

17. Cosmetic composition containing at least one citrus bioflavonoid and/or at least one bioflavonoid as defined in any one of Claims 1 to 4, characterized in that it contains, in addition, at least one reducing agent chosen from the alkali or alkaline-earth metal salts of metabisulphite, erythorbic acid and cysteine.

18. Composition according to Claim 17, characterized in that the reducing agent is chosen from metabisulphite salts and erythorbic acid.

19. Composition according to either of Claims 17 and 18, characterized in that the metabisulphite salts are sodium and potassium salts.

20. Composition according to any one of Claims 17 to 19, characterized in that the pH of the composition is less than 6.

21. Composition according to any one of Claims 17 to 20, characterized in that the pH of the composition is between 3 and 5.

22. Composition according to any one of Claims 17 to 21, characterized in that the reducing agent is present in concentrations ranging from 0.001 to 3% by weight relative to the total weight of the composition.

23. Composition according to Claim 22, characterized in that the reducing agent is present in concentrations ranging from 0.005 to 1% by weight relative to the total weight of the composition.

24. Composition according to any one of Claims 17 to 23, characterized in that the bioflavonoids are present at a concentration of between 0.001% and 10% and preferably between 0.005 and 5% by weight relative to the total weight of the composition.

25. Composition according to Claim 24, characterized in that the bioflavonoids are present at a concentration of between 0.005 and 5% by weight relative to the total weight of the composition.

26. Composition according to any one of Claims 17 to 25, characterized in that the cosmetic composition is as described in any one of Claims 6 to 16.

27. Cosmetic use according to any one of Claims 1 to 16, characterized in that the composition is as defined in any one of Claims 17 to 26.

## Patentansprüche

1. Kosmetische Verwendung mindestens eines Bioflavonoids aus Citrusfrüchten und/oder eines Bioflavonoids, das ausgewählt ist unter den Verbindungen der Formel (I), 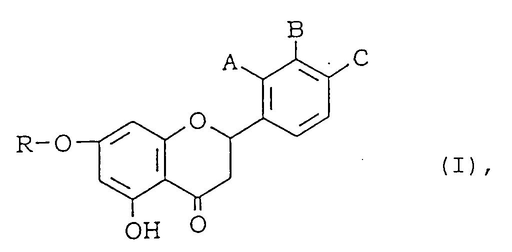 worin bedeuten:
- R eine Zuckergruppe,
- A Wasserstoff oder eine Alkoxygruppe mit 1 bis 4 Kohlenstoffatomen,
- B Wasserstoff, Hydroxy oder Alkoxy mit 1 bis 4 Kohlenstoffatomen und
- C Wasserstoff, Hydroxy oder Alkoxy mit 1 bis 4 Kohlenstoffatomen,
oder den Verbindungen der Formel (II), 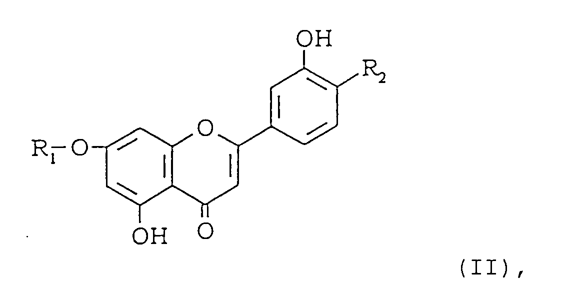 worin bedeuten: R₁ eine 6-O-(6-Desoxy-α-L-mannopyranosyl)-β-D-glucopyranosyl-Gruppe und
R₂ eine Alkoxygruppe mit 1 bis 4 Kohlenstoffatomen,
in einer kosmetischen Zusammensetzung als Behandlungsmittel zum Schutz oder zur Verbesserung der physikalischen Eigenschaften von Keratinmaterialien, insbesondere der mechanischen Eigenschaften und/oder der kosmetischen Eigenschaften.

2. Verwendung nach Anspruch 1, dadurch gekennzeichnet, daß A Wasserstoff oder Methoxy, B Wasserstoff, Hydroxy oder Methoxy, C Wasserstoff, Hydroxy oder Methoxy und R₂ Methoxy bedeuten.

3. Verwendung nach Anspruch 1 oder 2, dadurch gekennzeichnet, daß R eine Gruppe ist, die unter den Gruppen 6-O-(6-Desoxy-α-L-mannopyranosyl)-β-D-glucopyranosyl, 2-O-(6-Desoxy-α-L-mannopyranosyl)-β-D-glucopyranosyl und 6-Desoxy-α-L-mannopyranosyl ausgewählt ist.

4. Verwendung nach einem der vorhergehenden Ansprüche, dadurch gekennzeichnet, daß die Verbindung der Formel (I) unter Naringin, Neohesperidin, Hesperidin und Eriodictin ausgewählt ist.

5. Verwendung nach einem der vorhergehenden Ansprüche, dadurch gekennzeichnet, daß die Bioflavonoide in einer Konzentration im Bereich von 0,001 bis 10 Gew.-% und vorzugsweise von 0,005 bis 5 Gew.-%, bezogen auf das Gesamtgewicht der Zusammensetzung, vorliegen.

6. Verwendung nach einem der vorhergehenden Ansprüche, dadurch gekennzeichnet, daß die kosmetische Zusammensetzung in Form einer wäßrigen oder wäßrig-alkoholischen Lotion, als Gel, Creme, Emulsion, Vesikeldispersion, Schaum oder Spray vorliegt.

7. Verwendung nach einem der vorhergehenden Ansprüche, dadurch gekennzeichnet, daß die verbesserten kosmetischen Eigenschaften die Kämmbarkeit und/oder Frisierbarkeit und/oder die Weichheit sind.

8. Verwendung nach einem der Ansprüche 1 bis 7, dadurch gekennzeichnet, daß die verbesserten mechanischen Eigenschaften die Zugfestigkeit, die Bruchlast oder die Elastizität sind.

9. Verwendung nach einem der vorhergehenden Ansprüche, dadurch gekennzeichnet, daß die Keratinmaterialien Haare sind.

10. Verwendung nach einem der vorhergehenden Ansprüche, dadurch gekennzeichnet, daß die kosmetische Zusammensetzung eine Zusammensetzung für das Haar ist, die ausgespült oder nicht ausgespült wird oder auf dem Haar verbleibt.

11. Verwendung nach Anspruch 10, dadurch gekennzeichnet, daß die kosmetische Zusammensetzung für das Haar als Haarwaschmittel, Haarpflegemittel zur Anwendung nach der Haarwäsche, das ausgespült wird oder im Haar verbleibt, Dauerwellmittel, Mittel zur Entkräuselung, als Zusammensetzung zum Färben oder zum Entfärben oder auch als Haarspülung vorliegt, die zur Anwendung vor oder nach einer Färbung, einer Dauerwellbehandlung oder einer Entkräuselungsbehandlung oder auch zur Anwendung zwischen zwei Schritten einer Dauerwellbehandlung oder Entkräuselungsbehandlung vorgesehen ist.

12. Verwendung nach einem der Ansprüche 1 bis 8, dadurch gekennzeichnet, daß das Keratinmaterial Wimpern oder Augenbrauen sind.

13. Verwendung nach Anspruch 12, dadurch gekennzeichnet, daß die kosmetischen Zusammensetzungen Zusammensetzungen zur Pflege oder zum Schminken der Wimpern oder Augenbrauen sind.

14. Verwendung nach einem der Ansprüche 1 bis 8, dadurch gekennzeichnet, daß das Keratinmaterial Nägel sind.

15. Verwendung nach Anspruch 14, dadurch gekennzeichnet, daß die kosmetischen Zusammensetzungen Zusammensetzungen zur Pflege der Nägel oder Nagellacke sind.

16. Verwendung nach einem der vorhergehenden Ansprüche, dadurch gekennzeichnet, daß die kosmetische Zusammensetzung mindestens einen kosmetischen Hilfsstoff enthält.

17. Kosmetische Zusammensetzung, die mindestens ein Citrus-Bioflavonoid und/oder mindestens ein Bioflavonoid nach einem der Ansprüche 1 bis 4 enthält, dadurch gekennzeichnet, daß sie ferner mindestens ein Reduktionsmittel enthält, das unter den Alkali- oder Erdalkalimetabisulfiten, Erythorbinsäure und Cystein ausgewählt ist.

18. Zusammensetzung nach Anspruch 17, dadurch gekennzeichnet, daß das Reduktionsmittel unter den Metabisulfitsalzen und Erythorbinsäure ausgewählt ist.

19. Zusammensetzung nach Anspruch 17 oder 18, dadurch gekennzeichnet, daß die Metabisulfitsalze das Natriumsalz und das Kaliumsalz sind.

20. Zusammensetzung nach einem der Ansprüche 17 bis 19, dadurch gekennzeichnet, daß der pH-Wert der Zusammensetzung unter 6 liegt.

21. Zusammensetzung nach einem der Ansprüche 17 bis 20, dadurch gekennzeichnet, daß der pH-Wert der Zusammensetzung im Bereich von 3 bis 5 liegt.

22. Zusammensetzung nach einem der Ansprüche 17 bis 21, dadurch gekennzeichnet, daß das Reduktionsmittel in einer Konzentration im Bereich von 0,001 bis 3 Gew.-%, bezogen auf das Gesamtgewicht der Zusammensetzung, vorliegt.

23. Zusammensetzung nach Anspruch 22, dadurch gekennzeichnet, daß das Reduktionsmittel in einer Konzentration im Bereich von 0,005 bis 1 Gew.-%, bezogen auf das Gesamtgewicht der Zusammensetzung, vorliegt.

24. Zusammensetzung nach einem der Ansprüche 17 bis 23, dadurch gekennzeichnet, daß die Bioflavonoide in einer Konzentration im Bereich von 0,01 bis 10 Gew.-% und vorzugsweise von 0,005 bis 5 Gew.-%, bezogen auf das Gesamtgewicht der Zusammensetzung, vorliegen.

25. Zusammensetzung nach Anspruch 24, dadurch gekennzeichnet, daß die Bioflavonoide in einer Konzentration im Bereich von 0,005 bis 5 Gew.-%, bezogen auf das Gesamtgewicht der Zusammensetzung, vorliegen.

26. Zusammensetzung nach einem der Ansprüche 17 bis 25, dadurch gekennzeichnet, daß die kosmetische Zusammensetzung so vorliegt, wie in einem der Ansprüche 6 bis 16 beschrieben ist.

27. Kosmetische Verwendung nach einem der Ansprüche 1 bis 16, dadurch gekennzeichnet, daß die Zusammensetzung so vorliegt, wie in einem der Ansprüche 17 bis 26 beschrieben ist.
